# EUROPEAN PATENT SPECIFICATION

(11) **EP 0 717 847 B1**
(45) Date of publication and mention of the grant of the patent: **19.04.2000**
(21) Application number: 94927036.7
(22) Date of filing: 06.09.1994
(51) Int. Cl.: G01N 33/569, G01N 33/543, G01N 33/554

(54) **USE OF ANTIBODIES AGAINST BETA-2 MICROGLOBULIN-FREE, CLASS I HLA MOLECULES TO PROVIDE HIV DIAGNOSTIC TESTS AS WELL AS COMPOSITIONS HAVING A PROTECTIVE ACTION AGAINST HIV INFECTIONS**
VERWENDUNG VON ANTIKÖRPERN GEGEN BETA-2-MIKROGLOBULIN-FREIE KLASSE-I-HLA-MOLEKÜLEN ZUM ERHALT EINES DIAGNOSTISCHEN HIV-TESTS UND ZUSAMMENSETZUNGEN MIT SCHUTZWIRKUNG GEGEN HIV-INFEKTIONEN
UTILISATION D'ANTICORPS CONTRE DES MOLECULES HLA DE CLASSE I EXEMPTES DE BETA-2 MICROGLOBULINE AFIN DE PRODUIRE DES TESTS DE DIAGNOSTIC DU VIH AINSI QUE DES COMPOSITIONS A ACTION PROTECTRICE CONTRE DES INFECTIONS A VIH

(30) Priority: 10.09.1993 IT RM930613
(43) Date of publication of application: 26.06.1996
(73) Proprietor: FONDAZIONE CENTRO SAN RAFFAELE DEL MONTE TABOR, 20132 Milano (IT); Fondation Mondiale Recherche et Prevention SIDA, 75732 Paris Cédex 15 (FR)
(72) Inventor: BERETTA, Alberto, I-20132 Milano (IT)
(74) Representative: Ores, Irène
(86) International application number: IT9400146
(87) International publication number: WO9507465

(56) References cited:
- WO-A-93/23427
- JOURNAL OF EXPERIMENTAL MEDICINE, vol.174, no.7, July 1991, NEW YORK, NY, US pages 53 - 62 FABIO GRASSI ET AL. 'Human Immunodeficiency Virus Type 1 gp120 Mimics a Hidden Monomorphic Epitope Borne by Class I Major Histocompatibility Complex Heavy Chains' cited in the application
- JOURNAL OF INFECTIOUS DISEASES 168 (6). 1993. 1396-1403. ISSN: 0022-1899 DE SANTIS C ET AL 'Cross-reactive response to human immunodeficiency virus type 1 ( V -1) gp120 and HLA class I heavy chains induced by receipt of HIV -1-derived envelope vaccines.'
- MOL. IMMUNOL. (1992), 29(7-8), 989-98 CODEN: MOIMD5;ISSN: 0161-5890, 1992 WOLF, HANS ET AL 'Fine specificity of the murine antibody response to V -1 gp160 determined by synthetic peptides which define selected epitopes'
- J EXP MED 170 (6). 1989. 2023-2036. CODEN: JEMEAV ISSN: 0022-1007 TAKAHASHI H ET AL 'Structural Requirements for Class I MHC Molecule-Mediated Antigen Presentation and Cytotoxic T Cell Recognition of an Immunodominant Determinant of the Human Immunodeficiency Virus Envelope Protein.'
- J IMMUNOL 151 (4). 1993. 2283-2295. CODEN: JOIMA3 ISSN: 0022-1767 SHIRAI M et al 'Preferential V-Beta Usage by Cytotoxic T Cells Cross-Reactive between Two Epitopes of HIV-1 gp160 and Degenerate in Class I MHC Restriction.'

SEQUENCE LISTING

(1) GENERAL INFORMATION:

(i) APPLICANT:
(A) NAME: Fondazione Centro San Raffaele del Monte
Tabor
(B) STREET: Via Olgettina 60
(C) CITY: Milano
(E) COUNTRY: Italy
(F) POSTAL CODE (ZIP): 20132

(ii) TITLE OF INVENTION: Use of antibodies against beta2-
microglobulin free class I HLA molecules to provide HIV
diagnostic tests as well as compositions having a protective
action against HIV infection

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)


(2) INFORMATION FOR SEQ ID NO: 1:

(i) SEQUENCE CHARACTERISTICS:
(A) LENGTH: 50 amino acids
(B) TYPE: amino acid
(D) TOPOLOGY: linear

(ii) MOLECULE TYPE: peptide

(xi) SEQUENCE DESCRIPTION: SEQ ID NO: 1:

Asn Asn Gly Ser Glu Ile Phe Arg Pro Gly Gly Gly Asp Met Arg Asp
1               5                   10                  15

Asn Trp Arg Ser Glu Leu Tyr Lys Tyr Lys Val Val Lys Ile Glu Pro
            20                  25                  30

Leu Gly Val Ala Pro Thr Lys Ala Lys Arg Arg Val Val Gln Arg Glu
        35                  40                  45

Lys Arg
    50

(2) INFORMATION FOR SEQ ID NO: 2:

(i) SEQUENCE CHARACTERISTICS:
(A) LENGTH: 45 amino acids
(B) TYPE: amino acid

(D) TOPOLOGY: linear

(ii) MOLECULE TYPE: peptide

(xi) SEQUENCE DESCRIPTION: SEQ ID NO: 2:

Glu Pro Trp Val Glu Gln Glu Gly Pro Glu Tyr Trp Asp Arg Glu Thr
1               5                   10                  15

Gln Lys Tyr Lys Arg Gln Ala Gln Ala Asp Arg Val Asn Leu Arg Lys
            20                  25                  30

Leu Arg Gly Tyr Tyr Asn Gln Ser Glu Asp Gly Ser His
        35                  40                  45

(2) INFORMATION FOR SEQ ID NO: 3:

(i) SEQUENCE CHARACTERISTICS:
(A) LENGTH: 17 amino acids
(B) TYPE: amino acid
(D) TOPOLOGY: linear

(ii) MOLECULE TYPE: peptide

(xi) SEQUENCE DESCRIPTION: SEQ ID NO: 3:

Asp Pro Val Thr Pro Ala Glu Glu Ala Gly His Gln Lys Val Val Phe
1               5                   10                  15

Tyr

## Description

This invention relates to the use of antibodies against β-2 microglobulin-free, class I HLA molecules to provide tests to detect the acquired immunodeficiency virus (HIV), and protective compositions against HIV infections.

More specifically, this invention relates to diagnostic tests and compositions able to provide a passive immunity, to be used respectively in the AIDS diagnosis and preventive therapy.

The infection and the vaccination with HIV-I induce both a cellular and a humoral response (Lewis, D.E. et al., Int. Rev.Immunol., 7, 1-13, 1990; Tacket, C.Q. et al., AIDS Res. Hum. Retroviruses, 6, 535-542, 1990; Abrignani, S. et al., Proc. Natl. Acad. Sci. USA, 87, 6136-6140, 1990). However, the role of such responses has not explained yet, as to its opportunity to provide a protection against the virus infection. Antibodies anti-HIV are developed by HIV infected subjects and are able to inhibit the virus growth in vitro (Robert-Guroff, M. et al., Nature, 316, 72-74, 1985; HO, D.D. and al., J. Virol., 61, 2024-2028, 1987; Matthews, T.J. et al., Proc. Natl. Acad. Sci. USA, 83, 9709-9714, 1986). However, the induction of neutralizing antibodies in vitro in test animals by means of recombinant viral antigens is not associated with a protection against viral infection. A protection was obtained in the monkey/SIV virus experimental model, through an exposition to healthy cells (Stott, E., Nature, 353-393, 1991) associated with the presence of antibodies anti-cellular components, that are present in vaccines, showing that an immunity against non-viral antigens could be more effective than an immunity against viral antigens (Langlois, A.J. et al.,Science, 255, 292-293, 1992). However, target cells of such protective response were not identified yet.

Some recent studies found that HIV seronegative patients, with an high risk of HIV infection, had HIV-sensitized peripherial blood T lymphocytes (Clerici. M. et al., J. Inf. Diseases, 164, 178-182, 1991, Clerici. M. et al., J. Inf. Diseases, 165, 1012-1019, 1991), thus showing an infection resistant status, characterized in having a virus-specific cellular immunity, wherein no virus-specific antibodies are detected. This might suggest that virus-specific TH1 type T-helper cells could cooperate with other effector mechanisms, which, in turn, confer some protection against the infection. Therefore, seronegative patients, with an high risk of HIV infection, could theoretically be considered as protected against HIV infections.

Till now, no immunity, as detected in vaccinated animals, was found in such patients. On the contrary, antibodies against cellular antigens (HLA, Class I and II) were identified in HIV infected patients (Golding, H. et al., J. Exp. Med., 167, 914-923, 1988; Golding, H. et. al., J. Clin. Invest.,83, 1430-1435, 1989; Zaitzeva, M.B. et al., Scand. J. Immun., 35, 267-273, 1992). The author of the instant invention found that an immunization by means of recombinant proteins of the HIV envelope (gp 160) induced the development of antibodies against Class I HLA antigens (WO-A-93/23427). These antibodies detected epitopes of β2-microglobulin free HLA C heavy chains (HLA FHC), that are immunologically homologous to HIV-I gp160 epitopes. The epitopes are located within the HLA C α-1 domain, as well as in the gp120 C5 region. As Class I HLA antigens are directly involved in the antiviral immune response, as being able to present viral peptides to T cells, it might be suggested that antibodies anti-Class I HLA induced upon the immunization procedure exert a potentially dangerous side effect.

Cross-reactive antibodies against both Class I HLA and gp120 could be detected by means of specific synthetic peptides, or carrying out a competitive assay with monoclonal antibodies having the same binding specificity (Beretta, A. et al., Eur. J. Immunol., 17, 1793-1798, 1987; Grassi, F. et al., J. Exp. Med., 174, 362, 1991).

The author of the instant invention found that serum from seronegative patients, with a high risk of HIV infection, contained anti-HLA FHC antibodies. Therefore the author developed a detection test of HLA FHC specific antibodies for HIV seronegative subjects, with an high risk of HIV infection.

Antibodies having an analogous specifity to the one detected in such patients, could advantageously be used to provide compositions able to induce a passive HIV immunization. One of such useful antibodies is the M38 antibody, that could be obtained by immunizing BALB/c mice with HIV-I virions and assaying the supernatants of hybridoma cultures with uninfected monocyte cell lines (Beretta A. et al., Eur. J. Immunol., 17, 1793-1798, 1987). It must be understood that other antibodies having similar specifity can be used and are included in the scope of this invention. Such antibodies could be obtained by using as immunogens, peptides having sequences as described in WO-A-93/23427 and referred in Table 1 (HIV-26 or HLA-22 peptides), and screening hybridomas for the presence of antibodies, having a binding affinity to activated lymphocytes or to any HLA FHC expressing cell line.

Accordingly an object of the invention is the use of an antibody able to detect immunologically related epitopes of HIV gp160 protein and of β2-microglobulin free Class I HLA FHC, to provide a diagnostic test to detect the HIV infection in an individual which is negative for the presence of specific anti-HIV antibodies, said test being characterized in comprising the following steps:
- to incubate a sample obtained from said individual with a composition comprising HLA FHC molecules, pre-immobilized onto a solid carrier, under conditions allowing a sample-HLA FHC complex to form;
- to wash with a buffer under conditions allowing said complex not to dissociate;
- to incubate said complex with said antibody able to detect immunologically related epitopes of HIV gp160 protein and of β2-microglobulin free Class I HLA FHC under conditions allowing a sample-HLA FHC-antibody complex to form; and
- to detect said sample-HLA FHC-antibody complex.

Preferably, said HLA FHC molecules, pre-immobilized onto a solid carrier, comprise cultured cells expressing said HLA FHC molecules on the outher surface of the cell membrane.

Preferably said antibody able to detect both epitopes of HIV gp160 protein and of β2-microglobulin free Class I HLA FHC is obtained through an immunization with a peptide comprising one of the amino acid sequences of the HIV-26 or HLA-31 peptides.

It is also an object of this invention an immunological method to detect a HIV infection from an individual which is negative for the presence of specific anti-HIV antibodies, said method being characterized in comprising the steps as follows:
- to incubate a sample obtained from said individual with a composition comprising HLA FHC molecules, pre-immobilized onto a solid carrier, under conditions allowing a sample-HLA FHC complex to form;
- to wash with a buffer under conditions allowing said complex not to dissociate;
- to incubate said complex with a detecting system to form a complex sample-HLA FHC-detecting system; wherein said detecting system comprises an antibody that is able to detect immunologically related epitopes of HIV virus gp160 protein and of β2 microglobulin free Class I HLA FHC under conditions allowing a sample-HLA FHC-antibody complex to form;
- to detect said sample-HLA FHC-detecting system;
   or, alternatively:
- to incubate said sample obtained from said individual with a composition comprising at least one HIV gp160 epitope immunologically related to β2 microglobulin free Class I HLA FHC, pre-immobilized onto a solid carrier, under conditions allowing a sample-HIV gp160 epitope to form;
- to wash with a buffer under conditions allowing said complex not to dissociate;
- to incubate said complex with a detecting system to form a complex sample-HIV gp160 epitope-detecting system, wherein said detecting system comprises either an antibody, that is able to detect immunologically related epitopes of HIV virus gp160 protein and of β2 microglobulin free Class I HLA FHC under conditions allowing a sample-HIV gp160 epitope-antibody complex to form, or antibodies against human immunoglobulines;
- to detect said sample-HIV gp160 epitope-detecting system.

Preferably said HLA FHC molecules pre-immobilized on a solid carrier comprise cultured cells expressing said HLA FHC molecules on the outer surface of the cell membrane.

According to a preferred embodiment, said antibody is obtained through an immunization procedure using a peptide comprising one of the amino acid sequences of HIV-26 or HLA-22 peptides.

A further object of this invention is the use of an antibody able to detect immunologically related epitopes of HIV gp160 protein and of β2-microglobulin free Class I HLA FHC for the preparation of a composition which is able to provide a passive immunity against HIV infections.

Preferably said antibody is obtained by immunization using a peptide comprising one of the amino acid sequences of the HIV-26 or HLA-22 peptides.

This invention will be now described in some illustrating, but not limiting examples by reference to the annexed figures, wherein:
fig.1 is a cytofluorimetry profile of the G7 antibody on MT4 line (Dr. Martin Gullberg, Umea University, Sweden) as well as on peripheral lymphocytes of seronegative patients, that were activated by the SEB protein (PBMC-SEB);
fig.2 is an ELISA assay with the a G7 antibody on a viral lysate;
fig.3 is an inhibition of syncythia formation assay with the G7 antibody; and
fig.4 is an inhibition of syncythia formation assay with sera from HIV serum negative subjects with an high risk of HIV infection; numbers are to be related to Table 2.

### EXAMPLE 1 - Synthesis of peptides

Peptides were synthetized by means of an automatic synthesizer (Applied Biosystem, model ABT 431/A), according to the method of Merrifield, R.B., Science, 232, 341-347, 1986, and purified to homogenity through a reverse phase HPLC procedure before having been coupled to the solid phase. The amino acid analysis of 6N HCl hydrolized peptides was in agreement with expected values. Peptides used in the following tests had the sequences as shown in Table 1; sequences of gp 120-C5, HIAα-1 and ctrl-pep are reported also in the Sequence Listing as SEQ ID No. 1, No. 2 and No. 3, respectively. HIV-26 has the amino acid sequence from aa. 20 to aa. 45 of Seq ID No.1; HIV-31 has the amino acid sequence from aa. 30 to aa. 38 of Seq ID No.1; HLA-22 has the amino acid sequence from aa. 16 to aa. 37 of Seq ID No.2.

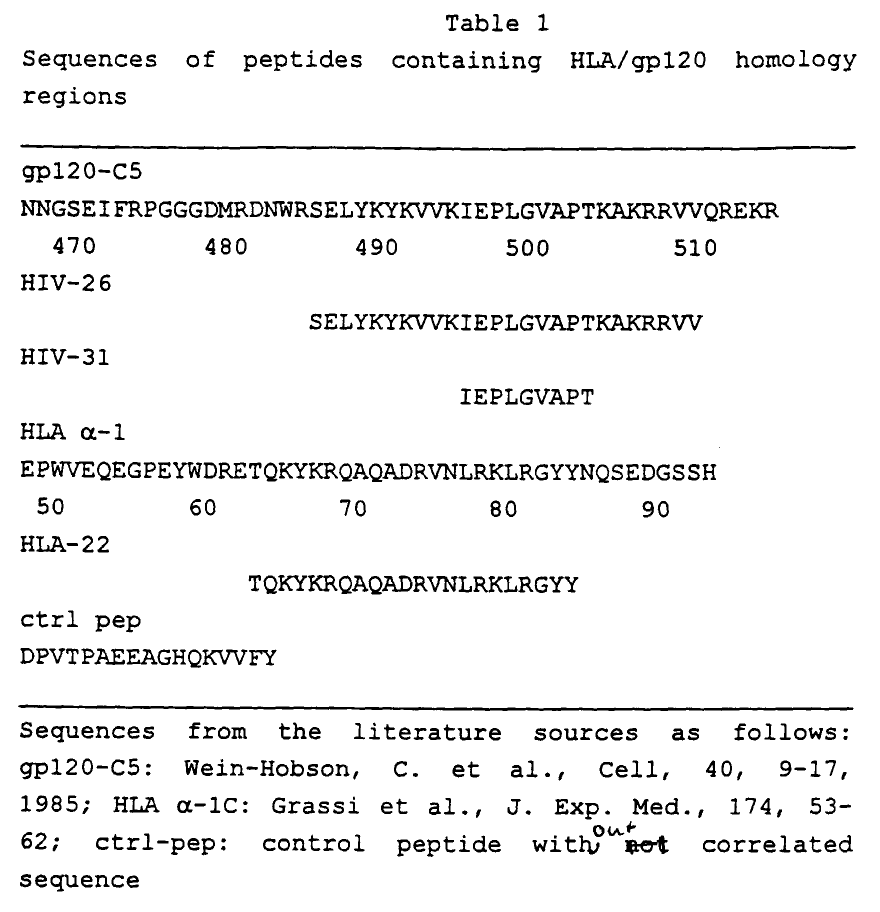

### EXAMPLE 2 - Production of G7 monoclonal antibody

A pair of female BALB/c mice were immunized 6 times at 15 day intervals through 10 µg of adjuvant-free HIV-26 peptide, by subcutaneous injections. After one month, animal sera were tested for the presence of the HIV-26 antibodies. A mouse showing a 1/2400 titer was sacrified and its spleen cells fused with the NS1 myeloma line, according to known methods. All the resulting clones were assayed for the presence of antibodies anti-HLA FHC through an ELISA assay with HLA-22 and HIV-31 peptides. The same clones were tested through an ELISA assay on microtitration trays, covered with HIV-I viral isolates.

In order to carry out the ELISA assays on peptides, 0.2 µg of Na₂CO₃/NaHCO₃ buffered peptides were used to fill all the wells of microtitration trays (Nunc, Denmark) for 3 hours at room temperature, followed by 1 hour saturation procedure with 5% of phosphate buffered BSA. Different serum dilutions, or a 50% diluted hybridoma supernatant solution in 3% phosphate buffered BSA, were then added, and the material incubated for 1 hour at 37°C, washed and tested to detect the reaction with peroxidase conjugated-rabbit anti-mouse immunoglobulins (Dakopatts, Denmark). Clones were positive when the absorbance was higher than the absorbance obtained when only the rabbit anti-mouse immunoglobulins were present.

In order to carry out the ELISA assays for HIV, HIV coated trays (Sorin Biomedica, Italy) were used. A 50% diluted hybridoma supernatant was added, the material was incubated for 1 hour at 37°C, washed and tested for the reaction with peroxidase conjugated rabbit anti-mouse immunoglobulins (Dakopatts, Denmark).

Positive clones in all three assays (HLA-22 and HIV-26 peptides and HIV viral lysates) were selected.

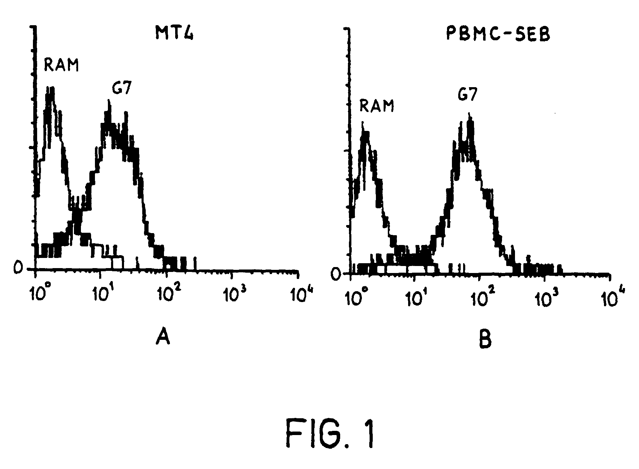

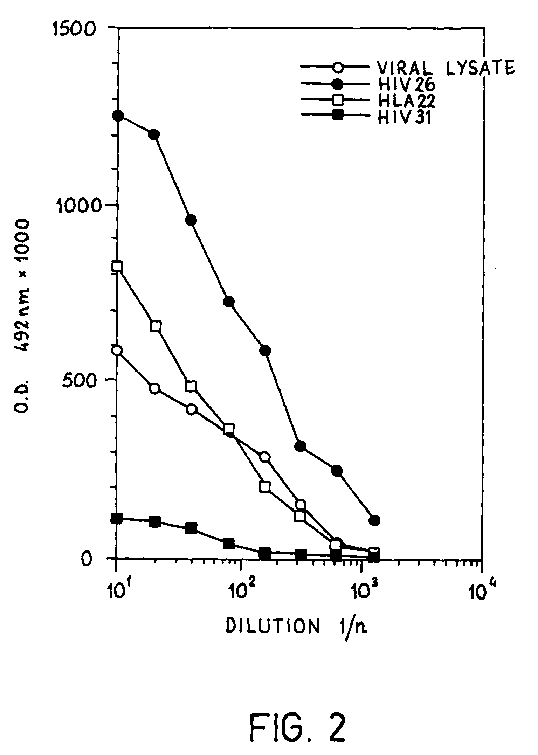

One of such clone (G7) was assayed through a flow cytometry procedure with the MT4 cell line and with peripheral blood T lymphocytes from a HIV negative donor, after activation using the SEB bacterial superantigen (Sigma Chemicals). SEB activated cells were obtained by T lymphocytes from peripheral blood cultures, separated by means of Ficoll Hypaque, in a RPMI medium for 3-5 days with 10% of bovine fetal serum and 1 µg/ml of SEB. Fig.1 shows the obtained pattern for the G7 antibody with the MT4 line through a flow cytometry procedure (A), and with SEB activated T lymphocytes from peripheral blood (B). Curves were very similar to those obtained for the other monoclonal antibodies anti-HLA FHC (M38 and L31). Fig. 2 shows an ELISA titration curve, that was obtained using the G7 antibody with three peptides, as in Table 1, as well as with an HIV-I viral lysate. The HIV-26 peptide contained the gp120 C5 epitopes, already identified as being homologous to HLA (see PCT Application No. IT/93/00049). The HIV-31 peptide contained the 9 amino acid sequence close to both of HLA homologous epitopes. The HLA-22 peptide contained the HLA C α-1 sequence, comprising both of the gp120 homologous regions. The G7 antibody was positive in respect of HIV-26 and HLA-22 peptides, but negative in respect of the HIV-31 peptide, as showed in respect of M38.

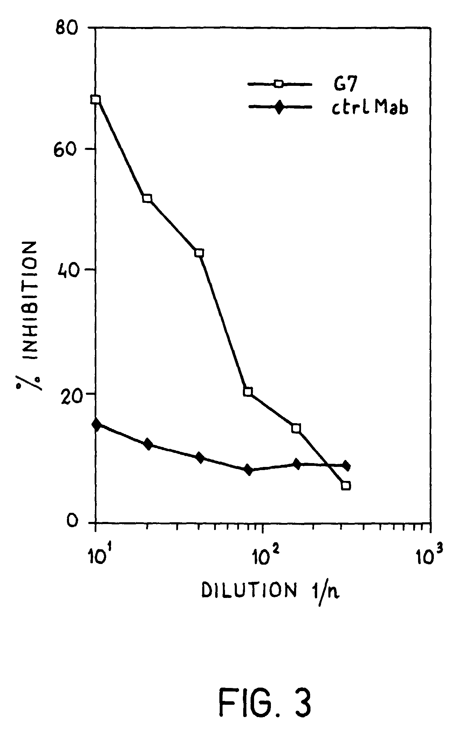

Fig.3 shows an inhibition of syncytium formation assay, as described in the Example 3, of the G7 antibody and control monoclonal antibodies of the same isotype (IgG γ-1), showing how the biological activity was depending upon the G7 dose.

The results demonstrated that, by immunizing with a synthetic peptide, monoclonal antibodies with the same specificity and biological activity as M38 were obtained.

### EXAMPLE 3 - Antibodies against HLA FHC as specific markers of HIV exposition and HIV inhibitors in vitro

A test to detect antibodies against HLA FHC through a flow cytometry procedure will be described herebelow.

The human lymphocyte line (MT4) was used in all tests. These lines expressed significant amounts of both HLA FHC and β2-microglobulin associated HLA heavy chains. Both M38 and G7 monoclonal antibodies are well known to provide a very similar immunofluorescence stain pattern on MT4 cells.

In order to detect serum antibodies directed against Class I HLA molecules, a 1/10 diluted serum to be analyzed was added to 5 x 10⁵ cells within a total volume of 0.5 ml of RPMI medium; the mixture was incubated for 30 minutes at 37°C, then washed with RPMI and incubated again with a 1/200 diluted M38 or G7 monoclonal antibody. After an incubation for 1 hour at 37°C, the cells were washed and incubated again with a 1/1000 diluted rabbit antiserum against mouse immunoglobulins in FITC (Sigma). The fluorescence was measured using a FacScan equipment (Becton Dickinson). Results were calculated as a percentage of the inhibition of the monoclonal antibody in presence of serum, according to the following formula: Channel average fluorescence of the monoclonal in the presence of serum, divided by the channel average fluoroscence of the monoclonal in the absence of serum, multiplied by 100. The test threshold value was calculated as the average value plus three standard deviations of the competitive percentage from 50 sera of HIV seronegative healthy blood donors. A sample was considered as positive for antibodies against HLA FHC when the obtained competitive percentage was higher than the threshold value. Threshold values are 5% and 20% for M38 and G7 antibodies respectively. The results are summarized in the Table 2.

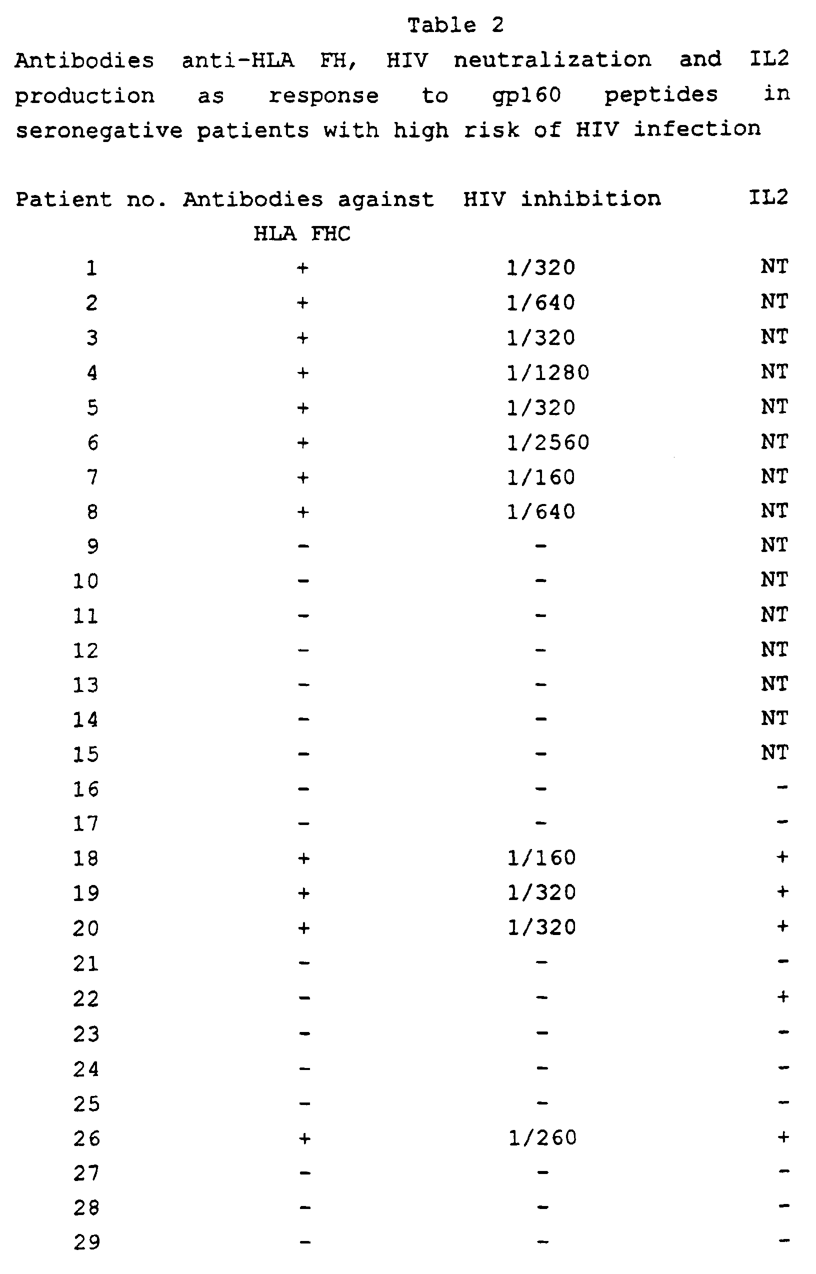

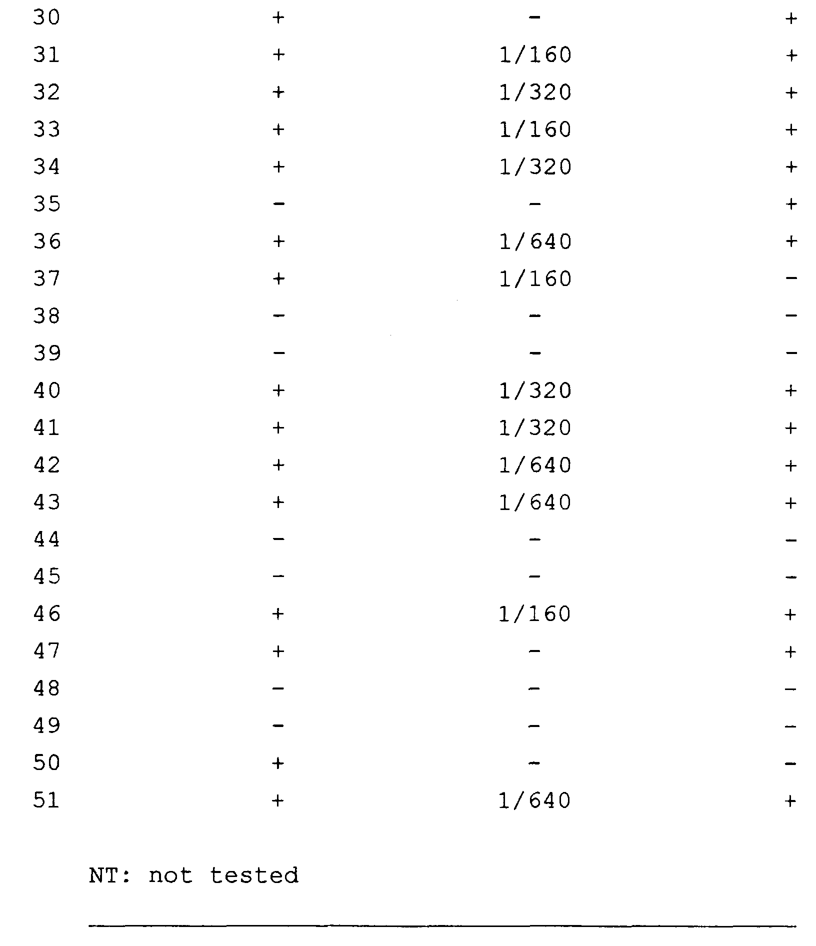

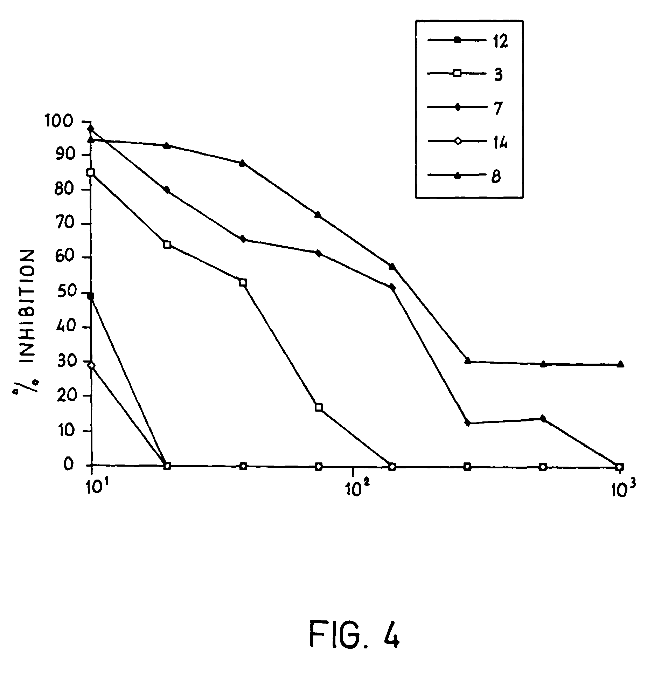

Sera were assayed for the presence of antibodies anti-HLA FHC and of anti-HIV neutralizing antibodies, through an inhibition assay. Briefly, sera were tested through a virus neutralizing assay in absence of cells, as described by Nara et al., AIDS Res. Hum. Retrovir., 3, 283-287, 1987; and Nara et al., Nature, 332, 469-471, 1988. 10⁶ CEM-SS cells (Aids Research & Reference Reagent Program Catalog cat. no. 776, NIH) were suspended in 25 µg of diethylamino ethyl dextrane (Pharmacia) for 30 minutes at 37°C and then loaded to microtitration trays, previously filled with 250 ng poly-L-lysine hydrobromide (Sigma, no.P-1399) and then incubated for 60 minutes at room temperature and washed. Approximatively 100 syncytium forming units of HIV-I IIIB (HTLV-III_{B}/H9, Aids Research & Reagent Reference Program Catalog, cat. no.398, NIH) or of HIV-I MN (HTLV-III_{MN}/H9, AIDS Res. & Reag. Ref. Program Catal, cat. no.317, NIH) were added in amount of 50 µl to each well; and an incubation for 5 days at 37°C was carried out. Antibodies and sera at different dilutions were added in amount of 150 µl. Syncytia were counted when 5 days were elapsed. Inhibiting titers were established by identifying the highest serum dilution enabling to produce a percentage of inhibition higher than 10%. HIV negative controls were carried out at each test. Results are reported in Table 2. In a first experiment, sera from seronegative patients, with an high risk of HIV infection, as sexual partners of seropositive subjects, were tested. Patients 1-15 were of this type. Eight of such patients (53%) resulted positive as for the presence of antibodies anti-HLA FHC, at least by using one of the monoclonal antibodies. The same patients also showed the presence of neutralizing antibodies anti-HIV. Fig.4 shows an inhibition of syncytium formation assay using sera from five of the tested patients. The sera 12 and 14 resulted negative for anti-HLA FHC, while sera 3, 7 and 8 were positive, thus showing that the presence of antibodies anti-HLA FHC was associated to a HIV inhibiting activity.

Accordingly, antibodies against HLA FHC were present in subjects with a high risk of HIV infection in a significant amount and their presence is associated with HIV neutralizing antibodies.

Only three patients showed the presence of specific antibodies for β2-microglobulin associated HLA FHC heavy chains, as shown by the competitive assay with the W632 monoclonal antibody (Barnstable, C.J. et al., Cell, 14, 9, 1978). None of such three samples showed an anti-HIV neutralizing activity. Accordingly, notwithstanding that specific monoclonal antibodies for β2-microglobulin associated, Class I HLA antigens inhibited HIV in vitro (Arthur, L.O., Science, 258, 1935-1938, 1992), antibodies having a similar specificity were neither detected within a significant percentage of subjects with high risk of HIV infection, nor were associated with HIV inhibition in vitro.

Further evidence that the presence of antibodies anti-HLA C should be a directly caused by an HIV direct exposition, was shown by the tests, carried out on 36 seronegative IVDU patients (16 to 51, Table 2), both for the presence of antibodies anti-HLA C in their sera and for the ability to produce IL2, after having been stimulated with HIV derived gp120 peptides. Clerici et al. (Clerici, M. et al., J. Inf. Diseases, 165, 51012-1019, 1991) demonstrated that subjects at high risk of HIV infection because of their sexual behaviour remained seronegative and had HIV-I sensitive T helper cells. The level of the specific sensitivity was established by assaying peripheral blood mononucleated cells for the IL2 production, as a response to amphipathic synthetic peptides, derived from the HIV-I envelope. Such findings showed that a cell mediated immune response could be detected before a seroconversion and the possibility to detect the HIV genome through polymerase chain reaction (PCR). Accordingly, a positive response to gp160 derived peptides through IL2 production in the peripheral blood of seronegative patients, would mean a HIV exposition risk.

Therefore the author analyzed sera from 36 seronegative drug consumers, already potentially exposed to HIV, for the presence of antibodies anti-HLA C and for the IL2 in vitro production as a gp160 peptide response, as described by Clerici, M. et al., J. Inf. Diseases, 165, 1012-1019, 1991. All sera were also tested for the presence of neutralizing antibodies against HIV.

Peripheral blood mononucleated cells from HIV serumnegative patients at high infection risk were obtained by heparinized blood, following a separation on Ficoll-Hypaque. 3 x 10⁵ cells were cultivated with RPMI medium, to which 5% of human serum was added. Synthetic peptides, sequences of which are referred by Clerici, M., et al., J. Inf. Diseases, 165, 1012- 1019, were added at 2.5 µM concentration. As a positive control for a proliferation response, the tetanus toxin antigen (Tetatox, Bern, Switzerland) was used at a concentration of 8-10 U/ml. As for IL2 production, the culture supernatants were picked up after 7 days and tested for the ability to induce a proliferation on a IL2 dependent cell line CTLL, by having loaded 8 x 10³ cells into each well of a microtitration tray with 96 wells. Cells were labelled with a pulse of 1 µCi of ³H-thymidine after 24 hours and blocked after 4 hours. A response was considered as positive, when cpms from the peptide stimulated, CTLL culture supernatants overcame the unstimulated control culture cpms. A positive response to two peptides at least was required to define any patient as positive.

The results are in the Table 2. Out of thirty-six tested patients (16 to 51), eigthteen (50%) were positive in both assays; fifteen (41%) were negative in both assays; and one was positive in respect of antibodies against HLA FHC and negative in respect of IL2 production; while the last two patients were positive as for the IL2 production and negative as for the antibodies against HLA FHC. Similar results occurred using both M38 and G7 antibodies. Out of nineteen sera, containing antibodies HLA FHC-anti, fifteen showed HIV neutralizing titers from 1/160 to 1/2560.

The results show a strong correlation between the presence of antibodies against HLA FHC in the sera and the ability of the peripheral blood T cells to give a response to gp120 peptides. Therefore, a HIV exposition induced either a gp160 specific response and a production of antibodies against HLA C in serumnegative subjects with an high risk of HIV infection.

As the antibodies against HLA FHC, that are present in HIV seronegative patients, show an in vitro activity against HIV, an antibody involvement in protecting against a HIV infection might be suggested and, consequently, immunoglobulin preparations for a HIV passive immunization made available.

The described anti-HLA FHC assay is quick, easy and customarily used, while the IL2 production assay requires a long time and specialized laboratories. The assay may be used to make tests on patients at high HIV infection risk or to test blood donors in order to avoid potentially HIV infected material.

## Claims

1. Use of an antibody able to detect immunologically related epitopes of HIV gp160 protein and of β2-microglobulin free Class I HLA FHC, in a diagnostic test to detect the HIV infection in an individual which is negative for the presence of specific anti-HIV antibodies, said test being characterized in comprising the following steps:
- to incubate a sample obtained from said individual with a composition comprising HLA FHC molecules, pre-immobilized onto a solid carrier, under conditions allowing a sample-HLA FHC complex to form;
- to wash with a buffer under conditions allowing said complex not to dissociate;
- to incubate said complex with said antibody able to detect immunologically related epitopes of HIV gp160 protein and of β2-microglobulin free Class I HLA FHC under conditions allowing a sample-HLA FHC-antibody complex to form; and
- to detect said sample-HLA FHC-antibody complex.

2. Use of an antibody able to detect immunologically related epitopes of HIV gp160 protein and of β2-microglobulin free Class I HLA FHC according to claim 1 wherein said HLA FHC molecules, preimmmobilized onto a solid carrier, comprise cultured cells expressing said HLA FHC molecules on the outer surface of the cell membrane.

3. Use of an antibody able to detect immunologically related epitopes of HIV gp160 protein and of β2-microglobulin free Class I HLA FHC according to claim 1 wherein said antibody able to detect immunologically related epitopes of HIV gp160 protein and of β2-microglobulin free Class I HLA FHC is obtained through an immunization with a peptide comprising one of the amino acid sequences:
HIV-26 : SELYKYKVVKIEPLGVAPTKAKRRVV
HIV-22 : TQKYKRQAQADRVNLRKLRGYY.

4. An immunological method to detect a HIV infection in an individual which is negative for the presence of specific anti-HIV antibodies, said method being characterized in comprising the steps as follows:
- to incubate a sample obtained from said individual with a composition comprising HLA FHC molecules, pre-immobilized onto a solid carrier, under conditions allowing a sample-HLA FHC complex to form;
- to wash with a buffer under conditions allowing said complex not to dissociate;
- to incubate said complex with a detecting system to form a complex sample-HLA FHC-detecting system; wherein said detecting system comprises an antibody that is able to detect immunologically related epitopes of HIV virus gp160 protein and of β2 microglobulin free Class I HLA FHC under conditions allowing a sample-HLA FHC-antibody complex to form;
- to detect said sample-HLA FHC-detecting system;
or, alternatively:
- to incubate said sample obtained from said individual with a composition comprising at least one HIV gp160 epitope immunologically related to β2-microglobulin free Class I HLA FHC, pre-immobilized onto a solid carrier, under conditions allowing a sample-HIV gp160 epitope to form;
- to wash with a buffer under conditions allowing said complex not to dissociate;
- to incubate said complex with a detecting system to form a complex sample-HIV gp160 epitope-detecting system, wherein said detecting system comprises either an antibody, that is able to detect immunologically related epitopes of HIV virus gp160 protein and of β2 microglobulin free Class i HLA FHC under conditions allowing a sample-HIV gp160 epitope-antibody complex to form, or antibodies against human immunoglobulins;
- to detect said sample-HIV gp160 epitope-detecting system.

5. An immunological method to detect a HIV infection from an individual which is negative for the presence of specific anti-HIV antibodies according to claim 4, wherein said HLA FHC molecules pre-immobilized on a solid carrier comprise cultured cells expressing said HLA FHC molecules on the outer surface of the cell membrane.

6. An immunological method to detect a HIV infection from an individual which is negative for the presence of specific anti-HIV antibodies according to claim 4 wherein said antibody is obtained through an immunization procedure using a peptide comprising one of the amino acid sequences of HIV-26 or HLA-22 peptides.

7. Use of an antibody able to detect immunologically related epitopes of HIV gp160 protein and of β2-microglobulin free Class I HLA FHC for the preparation of a composition which is able to provide passive immunity against HIV infections.

8. Use of an antibody able to detect immunologically related epitopes of HIV gp160 protein and of β2-microglobulin free Class I HLA FHC according to claim 7 wherein said antibody is obtained by immunization using a peptide comprising one of the amino acid sequences of the HIV-26 or HLA-22 peptides.

## Patentansprüche

1. Verwendung eines Antikörpers, welcher in der Lage ist, immunologisch verwandte Epitope des HIV-gp160-Proteins und der β2-Mikroglobulin-freien Klasse I HLA FHC nachzuweisen, in einem diagnostischen Test, um die HIV-Infektion in einem Individuum nachzuweisen, welches im Hinblick auf das Vorliegen von spezifischen anti-HIV-Antikörpern negativ ist, wobei der Test dadurch gekennzeichnet ist, daß er die folgenden Schritte umfaßt:
- Inkubieren einer Probe, die von dem Individuum erhalten wurde, mit einer Zusammensetzung, welche HLA FHC-Moleküle umfaßt, die auf einem festen Träger vorimmobilisiert wurden, unter Bedingungen, welche erlauben, daß sich ein Proben-HLA FHC-Komplex bildet;
- Waschen mit einem Puffer unter Bedingungen, welche nicht erlauben, daß der Komplex dissoziiert;
- Inkubieren des Komplexes mit dem Antikörper, welcher in der Lage ist, immunologisch verwandte Epitope des HIV-gp160-Proteins und der β2-Mikroglobulin-freien Klasse I HLA FHC nachzuweisen, unter Bedingungen, welche erlauben, daß sich ein Proben-HLA FHC-Antikörper-Komplex bildet; und
- Nachweisen des Proben-HLA FHC-Antikörper-Komplexes.

2. Verwendung eines Antikörpers, welcher in der Lage ist, immunologisch verwandte Epitope des HIV-gp160-Proteins und der β2-Mikroglobulin-freien Klasse I HLA FHC nachzuweisen, gemäß Anspruch 1, wobei die HLA FHC-Moleküle, welche auf einem festen Träger vorimmobilisiert wurden, kultivierte Zellen umfassen, welche die HLA FHC-Moleküle auf der äußeren Oberfläche der Zellmembran exprimieren.

3. Verwendung eines Antikörpers, welcher in der Lage ist, immunologisch verwandte Epitope des HIV-gp160-Proteins und der β2-Mikroglobulin-freien Klasse I HLA FHC nachzuweisen, gemäß Anspruch 1, wobei der Antikörper, welcher in der Lage ist, immunologisch verwandte Epitope des HIV-gp160-Proteins und der β2-Mikroglobulin-freien Klasse I HLA FHC nachzuweisen, durch eine Immunisierung mit einem Peptid erhalten wird, welches eine der folgenden Aminosäuresequenzen umfaßt:
HIV-26: SELYKYKVVKIEPLGVAPTKAKRRVV
HIV-22: TQKYKRQAQADRVNLRKLRGYY.

4. Ein immunologisches Verfahren, um eine HIV-Infektion in einem Individuum nachzuweisen, das im Hinblick auf das Vorliegen von spezifischen anti-HIV-Antikörpern negativ ist, wobei das Verfahren dadurch gekennzeichnet ist, daß es die folgenden Schritte umfaßt:
- Inkubieren einer Probe, die von dem Individuum erhalten wurde, mit einer Zusammensetzung, welche HLA FHC-Moleküle umfaßt, die auf einem festen Träger vorimmobilisiert wurden, unter Bedingungen, welche erlauben, daß sich ein Proben-HLA FHC-Komplex bildet;
- Waschen mit einem Puffer unter Bedingungen, welche nicht erlauben, daß der Komplex dissoziiert;
- Inkubieren des Komplexes mit einem Nachweissystem, um einen Komplex Probe-HLA FHC-Nachweissystem zu bilden; wobei das Nachweissystem einen Antikörper umfaßt, welcher in der Lage ist, immunologisch verwandte Epitope des HIV-gp160-Proteins und der β2-Mikroglobulin-freien Klasse I HLA FHC nachzuweisen, unter Bedingungen, welche erlauben, daß sich ein Proben-HLA FHC-Antikörper-Komplex bildet;
- Nachweisen des Proben-HLA FHC-Nachweissystems;
oder alternativ:
- Inkubieren der Probe, die von dem Individuum erhalten wurde, mit einer Zusammensetzung, welche wenigstens ein HIV-gp160-Epitop umfaßt, das immunologisch mit der β2-Mikroglobulin-freien Klasse 1 HLA FHC verwandt ist, welches auf einem festen Träger vorimmobilisiert wurde, unter Bedingungen, welche erlauben, daß sich ein Proben-HIV-gp160-Epitop(-Komplex) bildet;
- Waschen mit einem Puffer unter Bedingungen, welche nicht erlauben, daß der Komplex dissoziiert;
- Inkubieren des Komplexes mit einem Nachweissystem, um einen Komplex Probe-HIV-gp160-Epitop-Nachweissystem zu bilden, wobei das Nachweissystem entweder einen Antikörper, welcher in der Lage ist, immunologisch verwandte Epitope des HIV-Virus-gp160-Proteins und der β2-Mikroglobulin-freien Klasse I HLA FHC nachzuweisen, unter Bedingungen, welche erlauben, daß sich ein Proben-HIV-gp160-Epitop-Antikörper-Komplex bildet, oder Antikörper gegen menschliche Immunoglobuline umfaßt;
- Nachweisen des Proben-HIV-gp160-Epitop-Nachweissystems.

5. Ein immunologisches Verfahren, um eine HIV-Infektion eines Individuums nachzuweisen, das im Hinblick auf das Vorliegen von spezifischen anti-HIV-Antikörpern negativ ist, gemäß Anspruch 4, wobei die HLA FHC-Moleküle, die auf einem festen Träger vorimmobilisiert wurden, kultivierte Zellen umfassen, welche die HLA FHC-Moleküle auf der äußeren Oberfläche der Zellmembran exprimieren.

6. Ein immunologisches Verfahren, um eine HIV-Infektion eines Individuums nachzuweisen, das im Hinblick auf das Vorliegen von spezifischen anti-HIV-Antikörpern negativ ist, gemäß Anspruch 4, wobei der Antikörper durch ein Immunisierungsverfahren erhalten wird, welches ein Peptid verwendet, das eine der Aminosäuresequenzen der HIV-26- oder HLA-22-Peptide umfaßt.

7. Verwendung eines Antikörpers, welcher in der Lage ist, immunologisch verwandte Epitope des HIV-gp160-Proteins und der β2-Mikroglobulin-freien Klasse I HLA FHC nachzuweisen, zur Herstellung einer Zusammensetzung, welche in der Lage ist, eine passive Immunität gegenüber HIV-Infektionen zur Verfügung zu stellen.

8. Verwendung eines Antikörpers, welcher in der Lage ist, immunologisch verwandte Epitope des HIV-gp160-Proteins und der β2-Mikroglobulin-freien Klasse I HLA FHC nachzuweisen, gemäß Anspruch 7, wobei der Antikörper durch eine Immunisierung erhalten wird, wobei ein Peptid verwendet wird, welches eine der Aminosäuresequenzen der HIV-26- oder HLA-22-Peptide umfaßt.

## Revendications

1. Utilisation d'un anticorps capable de détecter des épitopes apparentés de façon immunologique de protéine gp160 de VIH et de HLA FHC de classe I exemptes de bêta-2-microglobuline, dans un test de diagnostic pour détecter l'infection à VIH chez un individu qui est négatif quant à la présence d'anticorps anti-VIH spécifiques, ledit test étant caractérisé en ce qu'il comprend les étapes suivantes :
- l'incubation d'un échantillon obtenu à partir dudit individu avec une composition comprenant des molécules HLA FHC, pré-immobilisées sur un support solide, dans des conditions qui permettent la formation d'un complexe échantillon-HLA FHC ;
- le lavage avec un tampon dans des conditions qui ne permettent pas la dissociation dudit complexe ;
- l'incubation dudit complexe avec ledit anticorps capable de détecter des épitopes apparentés de façon immunologique de protéine gp160 de VIH et de HLA FHC de classe I exemptes de bêta-2-microglobuline, dans des conditions qui permettent la formation d'un complexe échantilllon-HLA FHC-anticorps ; et
- la détection dudit complexe échantillon-HLA FHC-anticorps.

2. Utilisation d'un anticorps capable de détecter des épitopes apparentés de façon immunologique de protéine gp160 de VIH et de HLA FHC de classe I exemptes de bêta-2-microglobuline suivant la revendication 1, dans laquelle lesdites molécules HLA FHC, pré-immobilisées sur un support solide, comprennent des cellules cultivées exprimant lesdites molécules HLA FHC sur la surface extérieure de la membrane cellulaire.

3. Utilisation d'un anticorps capable de détecter des épitopes apparentés de façon immunologique de protéine gp160 de VIH et de HLA FHC de classe I exemptes de bêta-2-microglobuline suivant la revendication 1, dans laquelle ledit anticorps capable de détecter des épitopes apparentés de façon immunologique de protéine gp160 de VIH et de HLA FHC de classe I exemptes de bêta-2-microglobuline est obtenu par une immunisation avec un peptide comprenant l'une des séquences d'acides aminés :
VIH-26 : SELYKYKVVKIEPLGVAPTKAKRRVV
HLA-22 : TQKYKRQAQADRVNLRKLRGYY

4. Procédé immunologique pour détecter une infection à VIH chez un individu qui est négatif quant à la présence d'anticorps anti-VIH spécifiques, ledit procédé étant caractérisé en ce qu'il comprend les étapes suivantes :
- l'incubation d'un échantillon obtenu à partir dudit individu avec une composition comprenant des molécules HLA FHC, pré-immobilisées sur un support solide, dans des conditions qui permettent la formation d'un complexe échantillon-HLA FHC ;
- le lavage avec un tampon dans des conditions qui ne permettent pas la dissociation dudit complexe ;
- l'incubation dudit complexe avec un système de détection pour former un complexe échantilllon-HLA FHC-système de détection ; où ce système de détection comprend un anticorps qui est capable de détecter des épitopes apparentés de façon immunologique de protéine gp160 du virus VIH et de HLA FHC de classe I exemptes de bêta-2-microglobuline, dans des conditions qui permettent la formation d'un complexe échantillon-HLA FHC-anticorps ;
- la détection dudit complexe échantillon-HLA FHC-système de détection,
ou dans une variante :
- l'incubation dudit échantillon obtenu à partir dudit individu avec une composition comprenant au moins un épitope de gp160 de VIH apparenté de façon immunologique à des HLA FHC de classe I exemptes de bêta-2-microglobuline, pré-immobilisées sur un support solide, dans des conditions qui permettent la formation d'un complexe échantillon-épitope de gp160 de VIH ;
- le lavage avec un tampon dans des conditions qui ne permettent pas la dissociation dudit complexe ;
- l'incubation dudit complexe avec un système de détection pour former un complexe échantillon-épitope de gp160 de VIH-système de détection, où ce système de détection comprend soit un anticorps qui est capable de détecter des épitopes apparentés de façon immunologique de protéine gp160 du virus VIH et de HLA FHC de classe I exemptes de bêta-2-microglobuline, dans des conditions qui permettent la formation d'un complexe échantillon-épitope de gp160 de VIH-anticorps, soit des anticorps dirigés contre des immunoglobulines humaines ; et
- la détection dudit complexe échantillon-épitope de gp160 de VIH-système de détection.

5. Procédé immunologique pour détecter une infection à VIH chez un individu qui est négatif quant à la présence d'anticorps anti-VIH spécifiques suivant la revendication 4, dans lequel lesdites molécules HLA FHC pré-immobilisées sur un support solide, comprennent des cellules cultivées exprimant lesdites molécules HLA FHC sur la surface extérieure de la membrane cellulaire.

6. Procédé immunologique pour détecter une infection à VIH chez un individu qui est négatif quant à la présence d'anticorps anti-VIH spécifiques suivant la revendication 4, dans lequel ledit anticorps est obtenu par une procédure d'immunisation utilisant un peptide comprenant l'une des séquences d'acides aminés des peptides VIH-26 ou HLA-22.

7. Utilisation d'un anticorps capable de détecter des épitopes apparentés de façon immunologique de protéine gp160 de VIH et de HLA FHC de classe I exemptes de bêta-2-microglobuline pour la préparation d'une composition qui est capable de fournir une immunité passive contre des infections à VIH.

8. Utilisation d'un anticorps capable de détecter des épitopes apparentés de façon immunologique de protéine gp160 de VIH et de HLA FHC de classe I exemptes de bêta-2-microglobuline suivant la revendication 7, dans laquelle ledit anticorps est obtenu par immunisation avec un peptide comprenant l'une des séquences d'acides aminés des peptides VIH-26 ou HLA-22.
